# EUROPEAN PATENT SPECIFICATION

(11) **EP 1 277 054 B1**
(45) Date of publication and mention of the grant of the patent: **24.11.2004**
(21) Application number: 01931834.4
(22) Date of filing: 25.04.2001
(51) Int. Cl.: G01N 33/487, G01N 33/50, C12M 1/34

(54) **ELECTROCHEMICAL SENSING**
ELEKTROCHEMISCHES MESSGERÄT
DETECTION ELECTROCHIMIQUE

(30) Priority: 25.04.2000 GB 0009960
(43) Date of publication of application: 22.01.2003
(73) Proprietor: Oncoprobe Limited, Manchester M60 1QD (GB)
(72) Inventor: EDEN, Robert David, Warrington, Cheshire W13 4NW (GB); WOOLLEY, David Edward, Poynton, Cheshire SK12 1HU (GB)
(74) Representative: Allman, Peter John
(86) International application number: PCT/GB2001/001829
(87) International publication number: WO 2001/084142

(56) References cited:
- WO-A-96/15450
- WO-A-99/67628
- US-A- 4 225 410
- US-A- 4 963 245
- US-A- 6 010 889

## Description

The present invention relates to an electrochemical sensor and a method for monitoring signals generated by biological cells.

US Patent No. 6010889 describes a method and apparatus for assessing the metabolic and behavioural activities of viable cells by monitoring electrochemical signals generated by the cells. A culture medium incorporating cells of interest is introduced into a container so as to immerse the working electrode a portion of which is exposed within the container. The exposed portion of the electrode is covered with a thin film of gold. After the introduction of the culture medium a layer of adherent cells becomes established on the exposed working electrode surface. The working electrode communicates with a reference electrode via an arrangement within the container that provides electrochemical continuity. Detection circuitry is connected to monitor signals appearing between the working and reference electrodes, those signals being representative of processes occurring at the cell surfaces in contact with the working electrode. Analysis of the signals enables assessment of cell behaviour, for example the reaction of the cells to the introduction of a stimulant, suppressant or cytotoxic agent into the container.

The apparatus described in the above US patent functions satisfactorily but is inconvenient for large scale use, for example in comparative studies where several chemical agents or drugs are to be tested on the same cell culture in a speedy and economical manner. In particular, it is not easy to establish electrochemical continuity between a reference electrode and the culture medium within the container.

It is an object of the present invention to provide an improved electrochemical sensor and sensing method which may be applied to establish electrochemical continuity between a reference electrode and a body of culture medium, for example in the circumstances described in the above US patent.

According to the present invention, there is provided an electrochemical sensor comprising at least one first well for receiving a sample of culture medium containing biological cells, a working electrode a portion of which is exposed within the first well and is capable of supporting a layer of biological cells, and at least one second well located adjacent the first and separated therefrom by a wall, the wall being arranged such that, if the first well is filled with an electrochemically conductive culture medium to a first predetermined level sufficient to immerse the working electrode and the second well is filled with an electrochemically conductive culture medium to a second predetermined level, electrochemical continuity is established between the bodies of liquid within the first and second wells, whereby electrochemical signals generated by cells on the working electrode may be detected by monitoring signals generated between the working electrode and the liquid within the second well.

Preferably, a reference electrode is exposed within the or each second well below the second predetermined level. A single reference electrode may extend beneath an array of second wells. In some applications however electrical continuity between a reference electrode and liquid within the or each second well may be established by immersing the tip of an appropriately-supported reference electrode in that liquid rather than relying upon a reference electrode which is permanently incorporated into the sensor structure.

The invention also provides a method for monitoring signals generated by biological cells, wherein a layer of cells is formed on an exposed portion of a working electrode located within a first sensor well, the first well and an adjacent second well are filled to respective first and second predetermined levels with an electrochemically conductive culture medium, the wells being arranged such that electrochemical continuity is established between the bodies of culture medium in the first and second wells, and electrochemical cell activity is detected by monitoring signals appearing between the working electrode and a reference electrode in contact with culture medium in the second well.

The wall which separates the adjacent first and second wells can be arranged in various different ways to achieve the required electrochemical continuity between culture media within the two wells. For example, the wall may define an opening below the first and second predetermined levels such that bodies of culture medium filling the wells to the predetermined levels are in contact through the opening which may be for example a notch in the top edge of the wall. Alternatively, the wall may define an upper edge which is above the first and second predetermined levels, a liquid-absorbent material extending over the wall upper edge from below the first predetermined level in the first well to below the second predetermined level in the second well. Culture medium within the wells is absorbed into the material which extends over the upper edge of the wall, electrochemical continuity being achieved via the absorbed liquid. As a further alternative, the wall may define an upper edge above the first and second predetermined levels, the wall including an ion-permeable portion exposed in the first well beneath the first predetermined level and in the second well beneath the second predetermined level. The ion-permeable portion may be formed by adhesive or cement used to secure a lower edge of the wall partitioning to a base of the sensor assembly.

An array of first wells may be provided, each separated from a respective second well by a respective partitioning wall. Alternatively, an array of first wells may be provided with each of the first wells being separated from a common second well by a partitioning wall a portion of which allows electrochemical continuity between opposite sides of the wall.

A plurality of working electrodes may be spaced apart within each of the first wells to enable the comparison of signals generated from nominally identical layers of cells supported on each of the working electrodes within one well. To encourage the formation of cell layers on the working electrodes, exposed portions of the working electrodes may be located at the base of a recess defined by an opening in an insulating layer at the bottom of the first well. The working electrodes could be for example printed on a base board of the sensor assembly, the insulating layer being formed over the printed electrodes except for those portions which are to be exposed. Exposed portions could be given an appropriate electrically conductive surface treatment, for example a gold layer finish which is biocompatible with cell adhesion and growth. The gold layer could be applied to the working electrodes before the deposit of the insulating layer, openings in the insulating layer exposing small areas of the gold layer. Other surface treatments, which could be applied through openings in an insulating layer overlying the working electrodes, and which would facilitate cell attachment to improve the electrochemical signal, could include polylysine, antibodies, or the recognised matrix proteins fibronectin, gelatin, laminin or matrigel.

An embodiment of the present invention will now be described, by way of example, with reference.to the accompanying drawings, in which:
Figure 1 is a plan view of a sensor in accordance with the present invention;
Figure 2 is a perspective view of the sensor of Figure 1;
Figure 3 shows an alternative upper wall structure to that illustrated in Figures 1 and 2;
Figure 4 shows an alternative lower wall structure to that illustrated in Figures 1 and 2;
Figure 5 shows a side view of the wall structure of Figure 4; and
Figure 6 shows a further alternative upper wall structure.

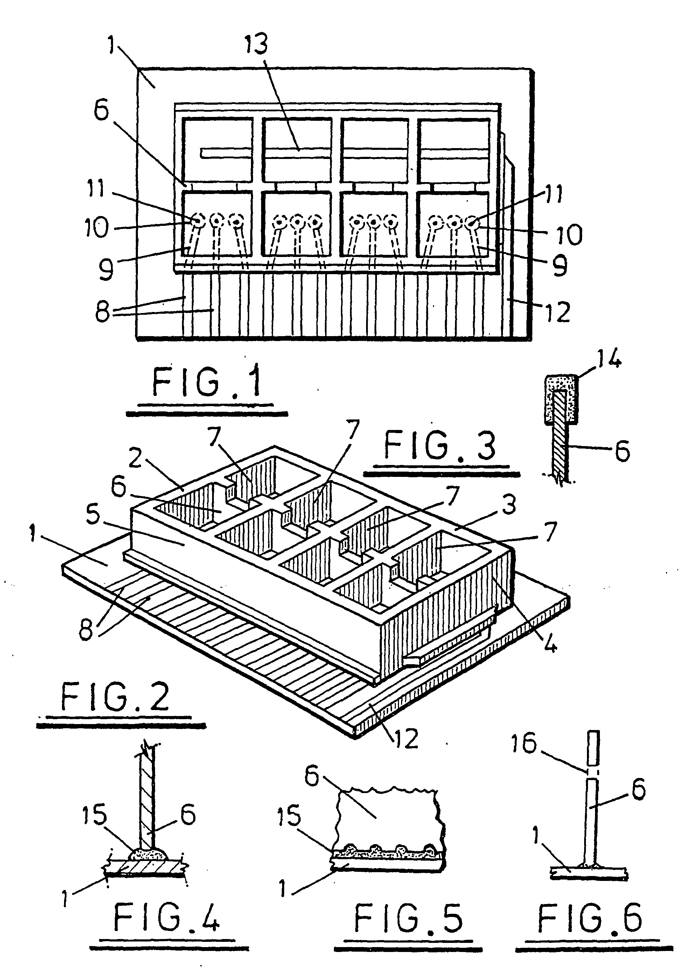

Referring to Figures 1 and 2, the illustrated sensor comprises a base board 1 of for example ceramic or plastic onto which is adhered a single piece body defining eight wells between a first side wall 2, a rear wall 3, a second side wall 4, a front wall 5 and a partition wall 6. Each of the eight wells is open at its base so that the upper surface of the base board 1 is exposed within the well. The partition wall 6 defines four notches 7 each of which is located between a respective pair of wells. Thus in each pair it can be considered that there is a first working well adjacent the front wall 5, and a second reference well adjacent the rear wall 3, an opening being defined between the working and reference wells by the notch 7 in the partition wall 6 formed between the working and reference wells.

The board 1 has printed upon its upper surface an array of twelve working electrode contact strips 8 of for example copper. Printing may be effected using a conventional screen printing technique. These strips have portions 9 which extend beneath each of the working wells and terminate in circular end portions 10. A layer of gold is electrodeposited onto the copper (usually on top of a layer of nickel). Alternatively gold could be vapour deposited. The printed board beneath each of the working wells is then covered by an electrically insulating layer defining apertures 11 which expose central areas of the end portions 10 of the electrodes at the bottom of the working wells. The electrically insulating layer could be formed for example using a printable ceramic. Such a material may be biocompatible and provide a suitable surface for attachment of the well-defining body. A contact strip 12 is also printed on the board 1, that contact strip 12 being continuous with a reference electrode 13 which extends across each of the four reference wells defined between walls 3 and 6 of the structure. The reference electrode 13 could be for example of silver/silver chloride. The strips, electrode surfaces and insulating layers may be formed in any convenient manner, for example using techniques used in the fabrication of printed circuit boards.

The board 1 and contact strips 8 and 12 are arranged such that the assembly can be plugged into a purpose built connector (not shown) so as to enable signals developed between any one of the working electrodes 11 and the common reference electrode 13 to be detected. In use, a culture medium would be introduced into each of the working wells so as to immerse the twelve electrodes 11. Biologically viable cells of interest would then be introduced into three of the four wells. The sensor would then be left undisturbed for a period sufficient for confluent cell layers to become established on the working electrodes. Confluent cell layers generate electrochemical signals which can be directly detected at the working electrode surface as described in US Patent No. 6010089, the cell layer in effect forming a barrier between the culture medium and the working electrode surface. Typically stable confluent cell layers will be established in a period of a few hours although it may be necessary to leave the cells undisturbed for a longer period, for example 24 hours, to enable the number of cells to increase as a result of cell division and to ensure that the cell layers have settled down.

The culture medium may be changed regularly, depending on cell type, and usually prior to any signal measurement or analysis, which may take place between several minutes to 48 hours after introduction of the culture medium. Fresh culture medium is introduced to a level just below that at which the culture medium would overflow from the working wells into the reference wells beneath which the reference electrode 13 extends.

Once the culture medium in the working wells has been carefully replaced, an aliquot of the same culture medium is introduced into the reference wells, immersing the electrode 13. Culture medium is introduced until it overflows the notches 7 in the partition walls 6, thereby establishing electrochemical continuity between the bodies of culture medium in the working and reference wells. Measurements of electrochemical activity at the surface of the working electrodes 11 can then be made by appropriate monitoring of signals generated between working electrodes 11 and the common reference electrode 12.

A working well filled with culture medium but into which no cells have been introduced provides a control with which the electrochemical signals generated in the other three working wells can be compared. Of the remaining three working wells, one may receive normal culture medium, another a cell stimulant, and the last could receive a cell suppressant The electrochemical signals are then compared between the cell free control, the cells to which neither a suppressant or stimulant has been supplied, the cells which have been stimulated, and the cells which have been suppressed. A rapid assessment of cell activity can accordingly be derived. A microscope examination of the working electrodes could also be made to visually assess all layers formed on the working electrode. This may be facilitated by arranging for the walls to be removable, for example by securing the walls to the base using a weak adhesive; the adherent cells being visualised by, for example, incident-fluorescence microscopy.

In the illustrated case, three independent working electrodes are included in each of the working wells to provide statistically reliable data which can be compared with signals generated from the control well and other test wells.

In the illustrated example, a common reference electrode 13 extends along each of the second or reference wells. It would of course be possible to have separate reference electrodes exposed within each of the reference wells. In addition, it would be possible to have a common single reference well rather than the four separate wells as illustrated in Figures 1 and 2.

In some uses of the embodiment of Figures 1 and 2, extraneous electrical fields in the vicinity of the assembly may be picked up, making it difficult to distinguish between such unwanted signals and for example electrochemical noise signals generated by cells forming confluent layers on the electrodes. To reduce such unwanted signal pick-up, signal processing circuits such as pre-amplifiers may be built directly on the base board 1.

In the embodiment of Figures 1 and 2 electrical continuity between bodies of culture medium in the working and reference wells is achieved by providing direct communication between the two bodies of medium across the notches 7 in the partition wall 6. Such an arrangement will result in some degree of mixing between the culture media in the two adjacent wells. Although this does not generally significantly affect the recorded signals, it is preferable to have the same culture media (and chemical or drug supplements) in the adjacent test and reference wells. Potential mixing of culture media could be prevented however if the notches 7 were filled by a material which would maintain electrochemical continuity, by allowing the exchange of ions and amino acids for example, but would prevent the exchange of larger molecules such as proteins, hormones, ligands or many synthetic drugs. A molecular weight cut off of for example 400 daltons would be appropriate.

Figure 3 illustrates an alternative arrangement for achieving electrochemical continuity between working and reference wells. Rather than relying upon a notch 7 in a partition wall 6, absorbent strip 14 extends over the top of the partition wall 6 so that the ends of the strip 14 would be immersed in the culture medium introduced into the working and reference wells. Culture medium absorbed into the strip 14 would then provide the necessary electrochemical continuity.

Figures 4 and 5 show an alternative arrangement for establishing electrical continuity between the working and reference wells. In the case of Figure 4, the partition wall 6 is secured to the base 1 by an adhesive 15, the adhesive 15 performing two functions, that is securing the wall 6 to the base 1 and providing a path for ion exchange whilst preventing leakage of culture medium and its larger sized constituents from the working to the reference well. As shown in Figure 5, the base of the working well/reference well partition wall may be serrated to facilitate electrochemical continuity between the two wells.

Figure 6 shows an alternative arrangement for achieving electrochemical continuity between working and reference wells. In this example, an opening 16 through the partition wall 6 is sized such that growth medium will wet the inner wall of the opening but not flow through because of the effect of surface tension. Culture medium introduced into the reference well will make an electrochemical connection with the meniscus formed within the partitioning wall opening.

When culture medium incorporating cells is left for a prolonged period to settle, cellular activity may change the pH value of the medium. To stabilise the pH value, the sensor may be maintained within a suitable controlled atmosphere, for example a 5% carbon dioxide plus air mixture, this being a conventional cell culture requirement.

In the embodiment of the invention illustrated in Figures 1 and 2, four pairs of working and reference wells are provided in a linear array. It will be appreciated that any convenient number of working cells with either respective reference cells, or a single common reference cell could be provided. It will also be appreciated that the working cells do not have to be arranged in a linear array. For example, a single common central reference well could be provided with working wells arranged there around, a single annular wall separating the working wells from the reference well. The single annular wall would be formed so as to enable the establishment of electrical continuity between a reference electrode immersed in a culture medium within the reference well and each of a series of bodies of culture medium in each of the working wells. A further well may also be provided to receive a representative sample of the cells under investigation, the further well having a relatively large exposed electrode surface structurally identical to each of the working electrode surfaces exposed in the normal working wells. The relatively large additional electrode surface could then be inspected using for example a microscope to assess the degree to which cells have established a confluent layer.

Although in the illustrated embodiment, three working electrodes are provided in each working well, it would be possible to provide only a single working electrode per working well or to provide two or more than three working electrodes per well.

The exposed surface area of each working electrode could be relatively small, for example a circular area of 0.5mm to 1.0mm diameter or less. The smaller the exposed area, the easier it will be to establish cell confluence. Although gold is the preferred surface metal for the working electrodes, other materials could be used, for example platinum. Platinum however is relatively responsive to fluctuations in pH value whereas the resting potential of gold electrodes appears to be almost unaffected by changes in temperature, serum protein and oxygen concentrations.

## Claims

1. An electrochemical sensor comprising at least one first well for receiving a sample of culture medium containing biological cells, a working electrode a portion of which is exposed within the first well and is capable of supporting a layer of biological cells, and at least one second well located adjacent the first and separated therefrom by a wall, the wall being arranged such that, if the first well is filled with an electrochemically conductive culture medium to a first predetermined level sufficient to immerse the working electrode and the second well is filled with an electrochemically conductive culture medium to a second predetermined level, electrochemical continuity is established between the bodies of liquid within the first and second wells, whereby electrochemical signals generated by cells on the working electrode may be detected by monitoring signals generated between the working electrode and the liquid within the second well.

2. A sensor according to claim 1, comprising a reference electrode exposed within the or each second well below the second predetermined level.

3. A sensor according to claim 2, comprising a plurality of second wells and a single reference electrode extending along the bottom of each second well.

4. A sensor according to claim 1, 2 or 3, wherein the wall defines an opening below the first and second predetermined levels such that bodies of liquid filling the wells to the predetermined levels are in contact through the opening.

5. A sensor according to claim 4, wherein the opening is defined by a notch formed in an upper edge of the wall.

6. A sensor according to claim 1, 2 or 3, wherein the wall defines an upper edge above the first and second predetermined wall levels, a liquid and ion-absorbent material extending over the wall upper edge from below the first predetermined level in the first well to below the second predetermined level in the second well to provide electrochemical continuity.

7. A sensor according to claim 1, 2 or 3, wherein the wall defines an upper edge above the first and second predetermined levels, the wall including an ion permeable portion exposed in the first well beneath the first predetermined level and in the second well beneath the second predetermined level.

8. A sensor according to claim 7, wherein the wall has a lower edge secured to a base by adhesive, the adhesive defining the ion-permeable portion of the wall.

9. A sensor according to any preceding claim, comprising an array of first wells each separated from a respective second well by a respective wall.

10. A sensor according to any one of claims 1 to 8, comprising an array of first wells each separated from a single common second well by a respective wall.

11. A sensor according to any preceding claim, wherein the or each first cell is provided with a plurality of working electrodes.

12. A sensor according to any preceding claim, wherein the or each exposed working electrode portion is located at the base of a recess defined by an opening in an insulating layer at the bottom of the first well.

13. A method for monitoring signals generated by biological cells, wherein a layer of cells is formed on an exposed portion of a working electrode located within a first sensor well, the first well and an adjacent second well are filled to respective first and second predetermined levels with an electrochemically conductive culture medium, the wells being arranged such that electrochemical continuity is established between the bodies of culture medium in the first and second wells, and electrochemical cell activity is detected by monitoring signals appearing between the working electrode and a reference electrode in contact with culture medium in the second well.

14. A method according to claim 13, wherein the layer of cells is formed by introducing cells into the first well in a culture medium, and leaving the culture medium in the first well until a layer of cells is formed over the exposed portion of the working electrode, and cell activity is detected by monitoring signals after filling the second well to the second predetermined level with the culture medium.

15. A method according to claim 14, wherein the layer of cells adheres to the exposed portion of the working electrode, and cell activity is detected by monitoring signals after the culture medium within the first well is removed and then replaced with fresh culture medium.

## Patentansprüche

1. Elektrochemischer Meßfühler, der aufweist: mindestens eine erste Vertiefung für das Aufnehmen einer Probe des Nährbodens, der biologische Zellen enthält; eine Arbeitselektrode, von der ein Abschnitt innerhalb der ersten Vertiefung freigelegt ist, und die in der Lage ist, eine Schicht von biologischen Zellen zu tragen; und mindestens eine zweite Vertiefung, die angrenzend an die erste angeordnet und davon durch eine Wand getrennt ist, wobei die Wand so angeordnet ist, daß, wenn die erste Vertiefung mit einem elektrochemisch leitenden Nährboden bis zu einem ersten vorgegebenen Niveau gefüllt ist, das ausreichend ist, um die Arbeitselektrode einzutauchen, und die zweite Vertiefung mit einem elektrochemisch leitenden Nährboden bis zu einem zweiten vorgegebenen Niveau gefüllt ist, eine elektrochemische Kontinuität zwischen den Flüssigkeitskörpem innerhalb der ersten und der zweiten Vertiefung hergestellt wird, wodurch elektrochemische Signale, die von Zellen an der Arbeitselektrode erzeugt werden, durch Überwachen der Signale nachgewiesen werden können, die zwischen der Arbeitselektrode und der Flüssigkeit innerhalb der zweiten Vertiefung erzeugt werden.

2. Meßfühler nach Anspruch 1, der eine Bezugselektrode aufweist, die innerhalb der oder jeder zweiten Vertiefung unterhalb des zweiten vorgegebenen Niveaus freigelegt wird.

3. Meßfühler nach Anspruch 2, der eine Vielzahl von zweiten Vertiefungen und eine einzelne Bezugselektrode aufweist, die sich längs des Bodens einer jeden zweiten Vertiefung erstreckt.

4. Meßfühler nach Anspruch 1, 2 oder 3, bei dem die Wand eine Öffnung unterhalb des ersten und des zweiten vorgegebenen Niveaus definiert, so daß die Flüssigkeitskörper, die die Vertiefungen bis zu den vorgegebenen Niveaus füllen, durch die Öffnung in Kontakt sind.

5. Meßfühler nach Anspruch 4, bei dem die Öffnung durch eine Kerbe definiert wird, die in einem oberen Rand der Wand gebildet wird.

6. Meßfühler nach Anspruch 1, 2 oder 3, bei dem die Wand einen oberen Rand über dem ersten und zweiten vorgegebenen Wandniveau definiert, wobei sich ein flüssiges und ionenabsorbierendes Material über den oberen Rand der Wand von unterhalb des ersten vorgegebenen Niveaus in der ersten Vertiefung aus nach unterhalb des zweiten vorgegebenen Niveaus in der zweiten Vertiefung erstreckt, um eine elektrochemische Kontinuität zu bewirken.

7. Meßfühler nach Anspruch 1, 2 oder 3, bei dem die Wand einen oberen Rand über dem ersten und zweiten vorgegebenen Niveau definiert, wobei die Wand einen ionendurchlässigen Abschnitt umfaßt, der in der ersten Vertiefung unterhalb des ersten vorgegebenen Niveaus und in der zweiten Vertiefung unterhalb des zweiten vorgegebenen Niveaus freigelegt wird.

8. Meßfühler nach Anspruch 7, bei dem die Wand einen unteren Rand aufweist, der an einer Basis mittels eines Klebstoffes gesichert wird, wobei der Klebstoff den ionendurchlässigen Abschnitt der Wand definiert.

9. Meßfühler nach einem der vorhergehenden Ansprüche, der eine Anordnung von ersten Vertiefungen aufweist, die jeweils von einer entsprechenden zweiten Vertiefung durch eine entsprechende Wand getrennt sind.

10. Meßfühler nach einem der Ansprüche 1 bis 8, der eine Anordnung von ersten Vertiefungen aufweist, die jeweils von einer einzelnen gemeinsamen zweiten Vertiefung durch eine entsprechende Wand getrennt sind.

11. Meßfühler nach einem der vorhergehenden Ansprüche, bei dem die oder jede erste Zelle mit einer Vielzahl von Arbeitselektroden versehen ist.

12. Meßfühler nach einem der vorhergehenden Ansprüche, bei dem der oder jeder freigelegte Arbeitselektrodenabschnitt an der Basis einer Aussparung angeordnet ist, die durch eine Öffnung in einer Isolationsschicht am Boden der ersten Vertiefung definiert wird.

13. Verfahren zum Überwachen von Signalen, die durch biologische Zellen erzeugt werden, bei dem eine Schicht aus Zellen auf einem freigelegten Abschnitt einer Arbeitselektrode gebildet wird, die innerhalb einer ersten Meßfühlervertiefung angeordnet ist, bei dem die erste Vertiefung und eine angrenzende zweite Vertiefung bis zum entsprechenden ersten und zweiten vorgegebenen Niveau mit einem elektrochemisch leitenden Nährboden gefüllt werden, wobei die Vertiefungen so angeordnet sind, daß zwischen den Körpern des Nährbodens in der ersten und zweiten Vertiefung eine elektrochemische Kontinuität hergestellt wird, und bei dem die elektrochemische Zellenaktivität durch Überwachen von Signalen nachgewiesen wird, die zwischen der Arbeitselektrode und einer Bezugselektrode in Kontakt mit dem Nährboden in der zweiten Vertiefung erscheinen.

14. Verfahren nach Anspruch 13, bei dem die Schicht aus Zellen durch Einführen von Zellen in die erste Vertiefung in einem Nährboden und Zurücklassen des Nährbodens in der ersten Vertiefung gebildet wird, bis eine Schicht aus Zellen über dem freigelegten Abschnitt der Arbeitselektrode gebildet wird, und bei dem die Zellenaktivität durch Überwachen von Signalen nach dem Füllen der zweiten Vertiefung bis zum zweiten vorgegebenen Niveau mit dem Nährboden nachgewiesen wird.

15. Verfahren nach Anspruch 14, bei dem die Schicht aus Zellen am freigelegten Abschnitt der Arbeitselektrode haftet, und bei dem die Zellenaktivität durch Überwachen von Signalen nachgewiesen wird, nachdem der Nährboden innerhalb der ersten Vertiefung entfernt und danach mit einem frischen Nährboden ersetzt wurde.

## Revendications

1. Capteur électrochimique comprenant au moins un premier puits pour recevoir un échantillon d'un milieu de culture contenant des cellules biologiques, une électrode de travail dont une partie est exposée dans le premier puits et est capable de supporter une couche de cellules biologiques, et au moins un deuxième puits agencé près du premier et séparé de celui-ci par une paroi, la paroi état agencée de sorte que lorsque le premier puits est rempli d'un milieu de culture à conduction électrochimique, jusqu'à un premier niveau prédéterminé suffisant pour immerger l'électrode de travail, le deuxième puits étant rempli d'un milieu de culture à conduction électrochimique jusqu'à un deuxième niveau prédéterminé, la continuité électrochimique est établie entre les corps de liquide dans les premier et deuxième puits, des signaux électrochimiques produits par les cellules sur l'électrode de travail pouvant ainsi être détectés en surveillant les signaux produits entre l'électrode de travail et le liquide dans le deuxième puits.

2. Capteur selon la revendication 1, comprenant une électrode de référence exposée dans le ou chaque deuxième puits au-dessous du deuxième niveau prédéterminé.

3. Capteur selon la revendication 2, comprenant plusieurs deuxièmes puits et une seule électrode de référence s'étendant le long du fond de chaque deuxième puits.

4. Capteur selon les revendications 1, 2 ou 3, dans lequel la paroi définit une ouverture au-dessous des premier et deuxième niveaux prédéterminés, de sorte que les corps de liquide remplissant les puits jusqu'aux niveaux prédéterminés sont en contact à travers l'ouverture.

5. Capteur selon la revendication 4, dans lequel l'ouverture est définie par une encoche formée dans un bord supérieur de la paroi.

6. Capteur selon les revendications 1, 2 ou 3, dans lequel la paroi définit un bord supérieur au-dessus des premier et deuxième niveaux de paroi prédéterminés, un liquide et un matériau à absorption d'ions s'étendant au-dessus du bord supérieur de la paroi, d'un point au-dessous du premier niveau prédéterminé dans le premier puits vers un point au-dessous du deuxième niveau prédéterminé dans le deuxième puits, pour assurer la continuité électrochimique.

7. Capteur selon les revendications 1, 2 ou 3, dans lequel la paroi définit un bord supérieur au-dessus des premier et deuxième niveaux prédéterminés, la paroi englobant une partie perméable aux ions exposée dans le premier puits au-dessous du premier niveau prédéterminé et dans le deuxième puits au-dessous du deuxième niveau prédéterminé.

8. Capteur selon la revendication 7, dans lequel la paroi comporte un bord inférieur fixé à une base par un adhésif, l'adhésif définissant la partie perméable aux ions de la paroi.

9. Capteur selon l'une quelconque des revendications précédentes, comprenant un ensemble de premiers puits, séparés chacun d'un deuxième puits respectif par une paroi respective.

10. Capteur selon l'une quelconque des revendications 1 à 8, comprenant un ensemble de premiers puits, séparés chacun d'un seul deuxième puits commun par une paroi respective.

11. Capteur selon l'une quelconque des revendications précédentes, dans lequel la ou chaque première cellule comporte plusieurs électrodes de travail.

12. Capteur selon l'une quelconque des revendications précédentes, dans lequel la ou chaque partie d'électrode de travail exposée est agencée au niveau de la base d'un évidement défini par une ouverture dans une couche isolante au fond du premier puits.

13. Procédé de surveillance de signaux produits par des cellules biologiques, dans lequel une couche de cellules est formée sur une partie exposée d'une électrode de travail agencée dans un premier puits du capteur, le premier puits et un deuxième puits adjacent étant remplis jusqu'à des premier et deuxième niveaux respectifs prédéterminés d'un milieu de culture à conduction électrochimique, les puits étant agencés de sorte à établir une continuité électrochimique entre les corps du milieu de culture dans les premier et deuxième puits, l'activité électrochimique des cellules étant détectée en surveillant les signaux apparaissant entre l'électrode de travail et une électrode de référence, en contact avec le milieu de culture dans le deuxième puits.

14. Procédé selon la revendication 13, dans lequel la couche de cellules est formée par introduction de cellules dans le premier puits dans un milieu de culture, le milieu de culture étant retenu dans le premier puits jusqu'à la formation d'une couche de cellules au-dessus de la partie exposée de l'électrode de travail, l'activité des cellules étant détectée en surveillant les signaux après le remplissage du deuxième puits au deuxième niveau prédéterminé du moyen de culture.

15. Procédé selon la revendication 14, dans lequel la couche de cellules adhère sur la partie exposée de l'électrode de travail, l'activité des cellules étant détectée en surveillant les signaux après l'enlèvement du milieu de culture contenu dans le premier puits et son remplacement par un milieu de culture frais.
